Europäisches Patentamt

European Patent Office

Office européen des brevets

(11) Veröffentlichungsnummer: **0 200 880**
**A2**

## (12) EUROPÄISCHE PATENTANMELDUNG

(21) Anmeldenummer: 86103064.1

(22) Anmeldetag: 07.03.86

(51) Int. Cl.⁴: **C07C 29/15** , C07C 31/04 , C01C 1/04

(30) Priorität: 27.04.85 DE 3515250

(43) Veröffentlichungstag der Anmeldung:
**17.12.86 Patentblatt 86/46**

(84) Benannte Vertragsstaaten:
**AT BE CH DE FR GB IT LI LU NL SE**

(71) Anmelder: **RUHRKOHLE AKTIENGESELLSCHAFT**
**Rellinghauser Strasse 1 Postfach 10 32 62**
**D-4300 Essen 1(DE)**
Anmelder: **Hoesch Aktiengesellschaft**
**Eberhardstrasse 12**
**D-4600 Dortmund 1(DE)**

(72) Erfinder: **Krüger, Horst, Dr.**
**Barbarastrasse 44**
**D-4330 Mülheim(DE)**
Erfinder: **Pelny, Hans**
**Borkumstrasse 17**
**D-4300 Essen(DE)**
Erfinder: **Neuenhahn, Peter, Dr.**
**Toblacher Weg 21**
**D-4352 Herten(DE)**
Erfinder: **Beier, Wolfgang**
**Augsburger Weg 71**
**D-4755 Holzwickede(DE)**

(54) Verfahren zur Herstellung von Chemierohstoffen.

(57) Nach der Erfindung werden Koksofengas und Hüttengas miteinander gemischt und in dieser Mischung direkt in einer Methanolsynthese eingesetzt.

EP 0 200 880 A2

## Verfahren zur Herstellung von Chemierohstoffen

Die Erfindung betrifft ein Verfahren zur Herstellung von Chemierohstoffen wie z.B. Methanol, Ammoniak aus Koksofengas und Hüttengas wie z.B. Konvetergas, wobei aus diesen Gasen zunächst ein stöchiometrisches Synthesegas erzeugt wird, das Koksofengas und Konvertergas in einem vorgegebenen Volumenverhältnis $H_2$ und CO gemischt und methanisiert werden, welches in einer Synthese zu Chemierohstoff wie z.B. Methanol und Ammoniak umgewandelt wird. In Stahlwerken, welche nach dem Blasstahlverfahren arbeiten, fällt Konvertergas an. Konvertergas wird in der Regel bisher nicht genutzt, dagegen Hochofengas. Häufig fällt jedoch überschüssiges Hochofengas an.

Konvertergas und überschüssiges Hochofengas bilden einen Rohstoff, den es zu nutzen gilt. Dazu bietet sich insbesondere die Methanol- und/oder Ammoniakherstellung an.

Für eine Methanolerzeugung aus Konvertergas allein liegt ein Kohlenstoffüberschuß vor. Um ein stöchiometrisches Synthesegas zu erhalten, muß das Konvertergas mit Wasserdampf konvertiert und der überschüssige Kohlenstaub über eine $CO_2$-Wäsche entfernt werden. Auf diese Weise kann Methanol mit einem thermischen Wirkungsgrad von nur 43 % erzeugt werden.

Beim Koksofengas ist eine ähnliche Situation wie beim Konvertergas und überschüssigen Hochofengas gegeben. Auch aus Koksofengas allein kann ein Synthesegas zur Methanolerzeugung bereitgestellt werden. Der hohe Wasserstoffüberschuß im Koksofengas führt zu einer relativ großen Purgegasmenge (Abblasegas aus der Methanolsynthese) bzw. Wärmemenge aus dem Purgegas, so daß über der Abdeckung des Wärmebedarfs der Prozeßanlage hinaus noch Dampf eportiert werden muß. Aus Koksofengas kann auf diese Weise Methanol mit einem thermischen Wirkungsgrad von 48 % hergestellt werden.

Sowohl der Wirkungsgrad von 43 % als auch der von 48 % eröffnen noch keine wirtschaftliche Methanolherstellung.

In einer japanischen Patentanmeldung, JP-56-8408 vom 22.01.1982, wurde vorgeschlagen, Koksofengas (COG) und Konvertergas (LDG) im Volumenverhältnis $H_2$ und CO von 3 zu mischen und dann zu methanisieren. Das methanreiche Gas wird nach weiterer Aufbereitung durch Sauerstoffzugabe an einem CrO-Katalysator partiell zu Methanol oxidiert.

Der Erfindung liegt daher die Aufgabe zugrunde, die Wirtschaftlichkeit der Methanolherstellung aus Konvertergas und überschüssigem Hochofengas bzw. aus Koksofengas zu verbessern. Dabei geht die Erfindung von dem japanischen Vorschlag eines Verbundes zwischen Konvertergas bzw. Hochofengas und Koksofengas aus.

Abweichend von dem bisher Bekannten wird nun vorgeschlagen, Koksofengas bzw. aufbereitetes Koksofengas mit Konvertergas im Volumenverhältnis $H_2$ und CO von ca. 2 zu mischen und dann direkt in einer Methanolsynthese einzusetzen.

Durch diese Mischung von Konvertergas mit hohem CO-Anteil und Koksofengas mit hohem Wasserstoffgehalt können die energieverbrauchenden Anlageteile, wie z.B. die CO-Konvertierung und der Steamreformer, erheblich verkleinert werden bzw. ganz entfallen. Aus der Mischung von Koksofengas und Konvertergas kann man nach der Erfindung Methanol mit einem thermischen Wirkungsgrad von über 60 % herstellen.

Für die Wasserstofferzeugung aus Koksofengas können Trenn-und Spaltverfahren eingesetzt werden.

Reine Trennverfahren, die lediglich durch physikalische Verfahrensschritte den hohen Wasserstoffanteil aus dem Koksofengas abtrennen, sind die Druckwechseladsorption (DWA) und die Tieftemperaturzerlegung.

In dem Spaltverfahren-Steamreforming und partielle Oxidation, werden zusätzlich, vor oder nach Abtrennung des Wasserstoffes, die Kohlenwasserstoffe des Koksofengases umgesetzt und damit die Wasserstoffausbeute erhöht.

Prinzipiell sind alle Trenn-und Spaltverfahren für die Aufbereitung des Koksofengases einsetzbar. Insbesondere wirtschaftliche Gründe sprechen jedoch für eine Kombination von DWA und Steamreforming.

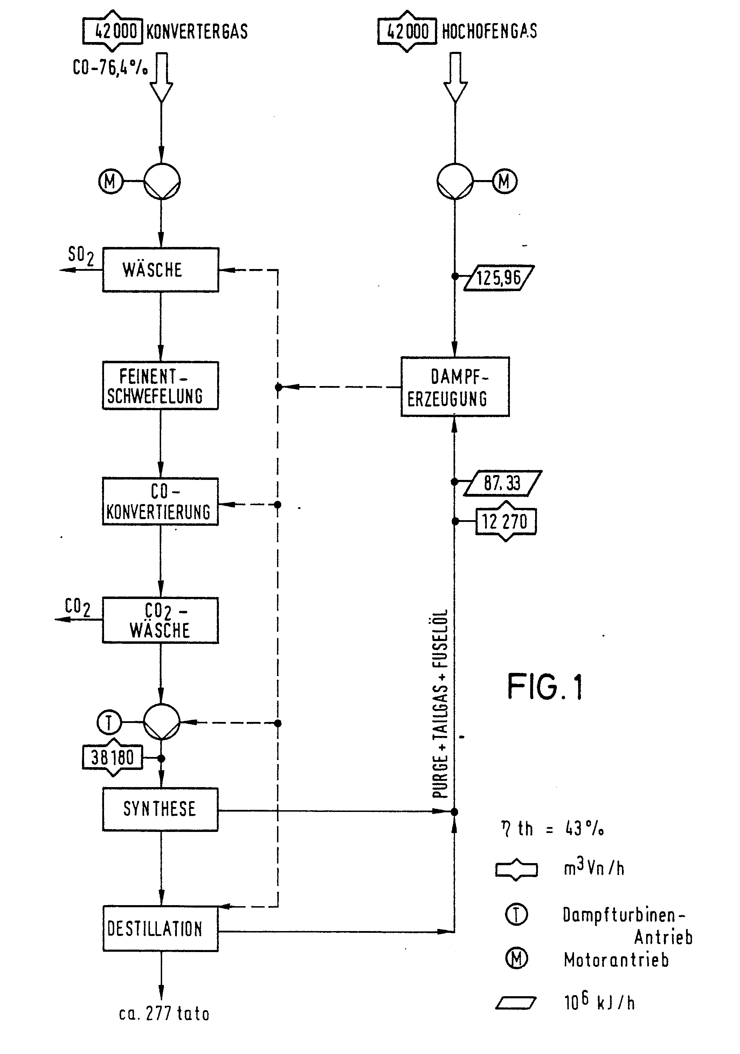

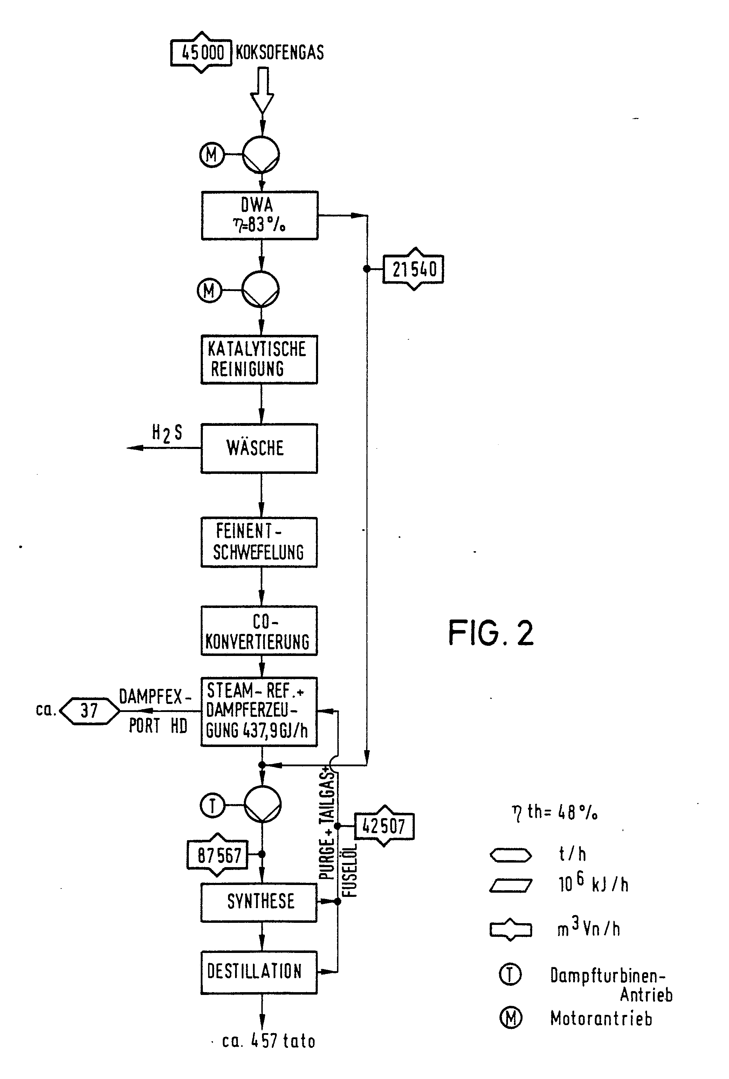

Die Herstellung von Methanol aus Konvertergas ist in Abb. 1, die Herstellung von Methanol aus Koksofengas in Abb. 2 dargestellt.

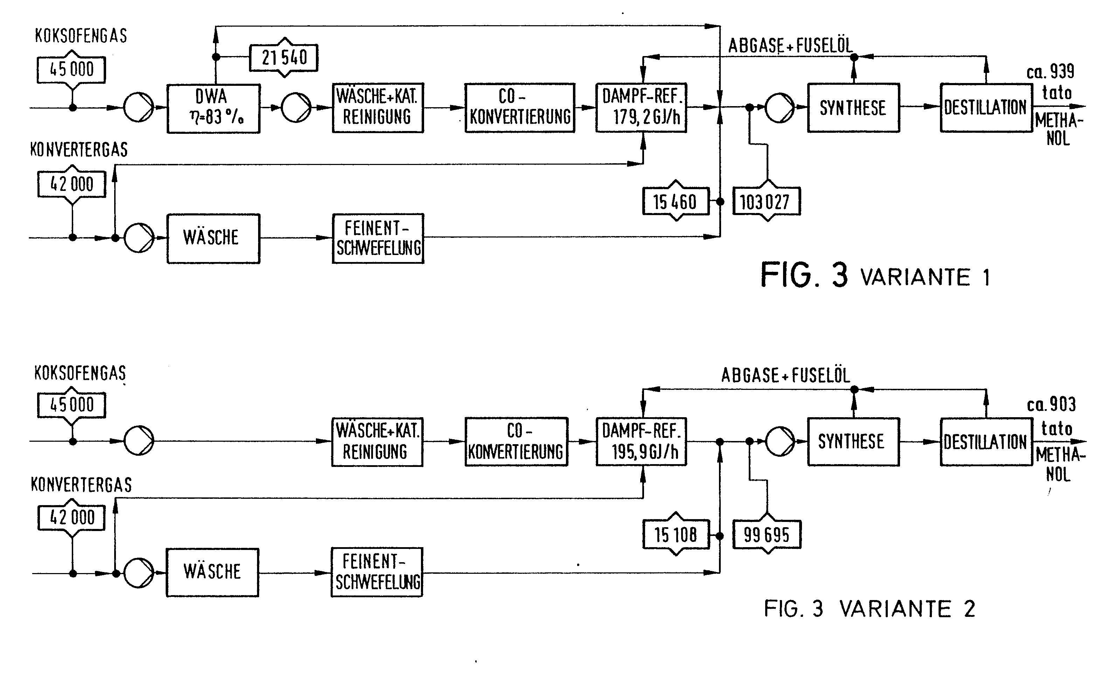

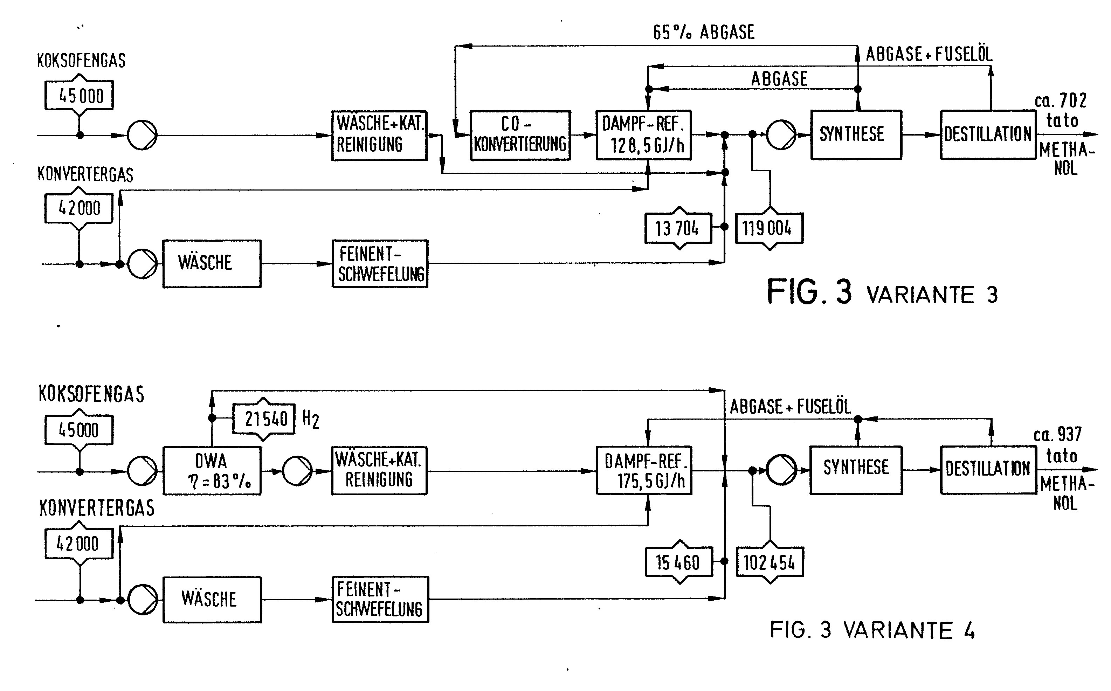

Abb. 3 zeigt 4 Verfahrensvarianten zur Aufbereitung von Koksofengas und Konvertergas zur Herstellung eines Methanol-Synthesegases.

Variante 1

Das an der Anlagengrenze zur Verfügung stehende Koksofengas wird nach Verdichtung über eine Druck-Wechsel-Anlage (DWA) geleitet, in der ca. 83 % des im Koksofengas enthaltenen Wasserstoffs abgetrennt werden.

Das Abgas der DWA wird erneut verdichtet und anschließend in einer Reinigungsstufe (Wäsche + katalytische Reinigung) von Katalysatorgiften befreit. Das so gereinigte Gas wird konvertiert, um Kohlenstoffablagerungen im folgenden Dampf-Reformer zu vermeiden und so eine Schädigung des Katalysators zu verhindern. Im Dampfreformer wird das im Gas enthaltene Methan mit Hilfe von Wasserdampf zu CO, $CO_2$ und $H_2$ aufgespalten.

Das Spaltgas wird mit dem in der DWA-Anlage abgetrennten Wasserstoff und mit einer zur Erzeugung eines stöchiometrischen Synthesegases erforderlichen Mengen Konvertergas, welches vorher verdichtet und gereinigt (Wäsche und Feinentschwefelung zur Entfernung der Katalysatorgifte) wurde, gemischt.

Anschließend wird des Synthesegas auf Synthesedruck verdichtet und in der Methanolsynthese und Destillation zu der gewünschten Produktqualität aufgearbeitet.

Die zur Spaltung des DWA-Abgases im Reformer benötigte Wärmemenge wird durch Abgas aus Synthese und Destillation sowie überschüssiges Konvertergas gedeckt.

Die Produktion beträgt bei den an der Anlagengrenze zur Verfügung stehenden Mengen von 45 000 m³/h (Vn) Koksofengas und 42 000 m³/h (Vn) Konvertergas ca. 939 tato Methanol.

Variante 2

Die Variante 2 unterscheidet sich im Vergleich zu Variante 1 nur dadurch, daß der im Koksofengas enthaltene Wasserstoff nicht über eine DWA-Anlage abgetrennt wird.

Bei gleichen Einsatzstoffen an Koksofengas und Konvertergas ergibt sich eine Produktion von 903 tato Methanol. Diese Verringerung der Produktion im Vergleich zu Variante 1 ist darauf zurückzuführen, daß sich durch die vorherige Abtrennung des Wasserstoffs bessere Gleichgewichtsverhältnisse im Dampfreformer einstellen, was im Endeffekt zu einer höheren Synthesegasmenge führt.

Außerdem ist die Varinate 1 durch die DWA flexibler z.B. beim Ausfall des Konvertergases, da hierdurch der Wasserstoffüberschuß in Synthesegas gemindert werden kann (höhere Produktion).

Variante 3

Bei dieser Variante wird das Koksofengas nach Verdichtung zunächst von Katalysatorgiften in einer Reinigungsstufe (Wäsche und Kat. Reinigung) befreit. Anschließend wird es mit Konvertergas, das vorher komprimiert und in Reinigungsstufen (Wäsche und Feinentschwefelung) von Katalysatorgiften befreit wurde, sowie Spaltgas aus dem Dampfreformer gemischt.

Bei dem Einsatzgas für den Dampfreformer handelt es sich um 65 % des Abblasegases (Methan reich) aus der Synthese, das vor der Dampfreformierung noch konvertiert wird, um Kohlenstoffablagerungen im Dampfreformer zu vermeiden.

Das stöchiometrische Gasgemisch aus Konvertergas, Koksofengas und Spaltgas wird auf Synthesedruck verdichtet und in der Methanolsynthese und Destillation auf die gewünschte Produktqualität aufgearbeitet.

Zur Beheizung des Dampfreformers wird überschüssiges Konvertergas, das restliche Abgas aus der Synthese sowie der Destillation eingesetzt.

Die Produktion beträgt bei dieser Variante ca. 702 tato Methanol. Grund für diese niedrige Produktion im Vergleich zu Variante 1 und 2 ist der hohe Inertengehalt im Synthesegas, der in Form von Methan aus dem Koksofengas herrührt.

Variante 4

Die Variante 4 ist bis auf die CO-Konvertierung mit der Variante 1 identisch.

Es zeigt sich, daß die Konvertierung praktisch keinen Einfluß auf die Endproduktion hat. Es wurde jedoch von mehreren Dampfreformer-Katalysator-Herstellern eine CO-Konvertierung empfohlen, um Kohlenstoffablagerungen zu verhindern.

Methanolerzeugung im Energieverbund Hütte-Kokerei

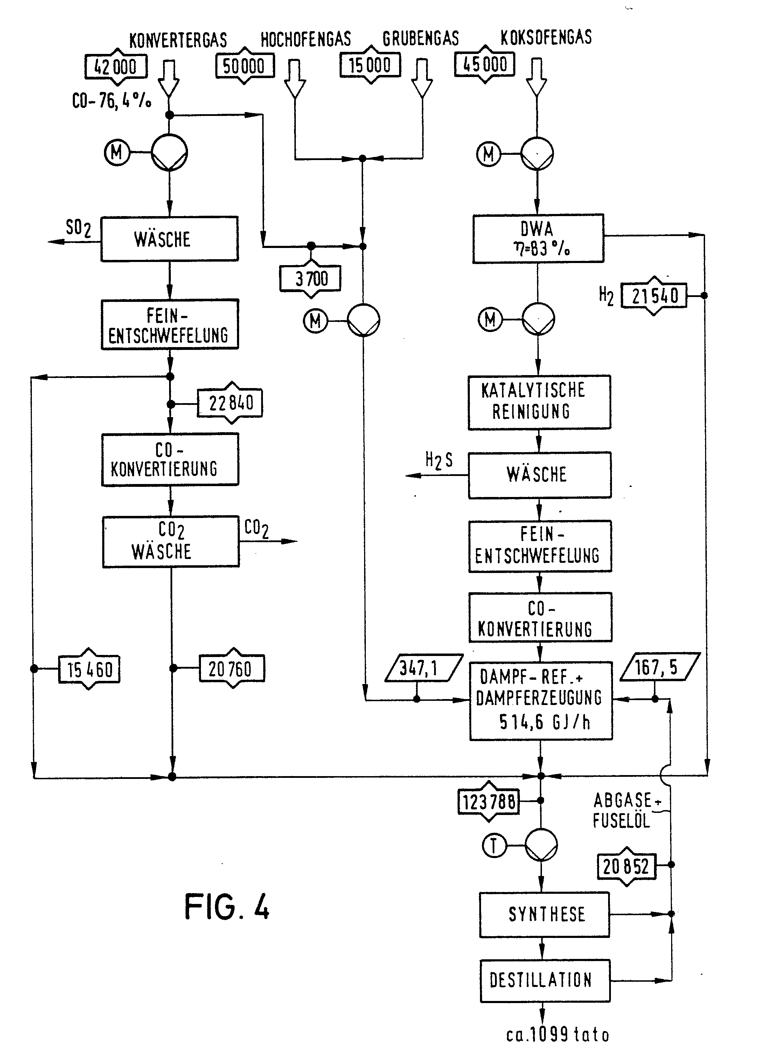

Den bisher untersuchten Verfahrensvarianten liegt eine Anlagenkonzeption zugrunde, bei der nur Koksofengas und Konvertergas als Rohstoff und als Energie eingesetzt werden. In einem Verbund Hütte-Kokerei kann jedoch noch auf andere Gase, wie z.B. Hochofengas und Grubengas, zurückgegriffen werden; diese Gase können als Heizenergie eingesetzt werden und damit kann mehr bisher verbranntes Konvertergas für die Methanolsynthese zur Verfügung gestellt werden (Abb. 4). Um ein stöchiometrisches Gasgemisch aus Koksofengas und Konvertergas für die Methanolsynthese zu erreichen, muß jedoch ein erheblicher Anteil des Konvertergases mit Wasserdampf über ein CO-Konvertierung in Wasserstoff umgewandelt werden. Durch die Zusatzenergie kann die Methanolproduktion auf ca. 1099 tato gesteigert werden. Der thermische Wirkungsgrad beträgt 61 %.

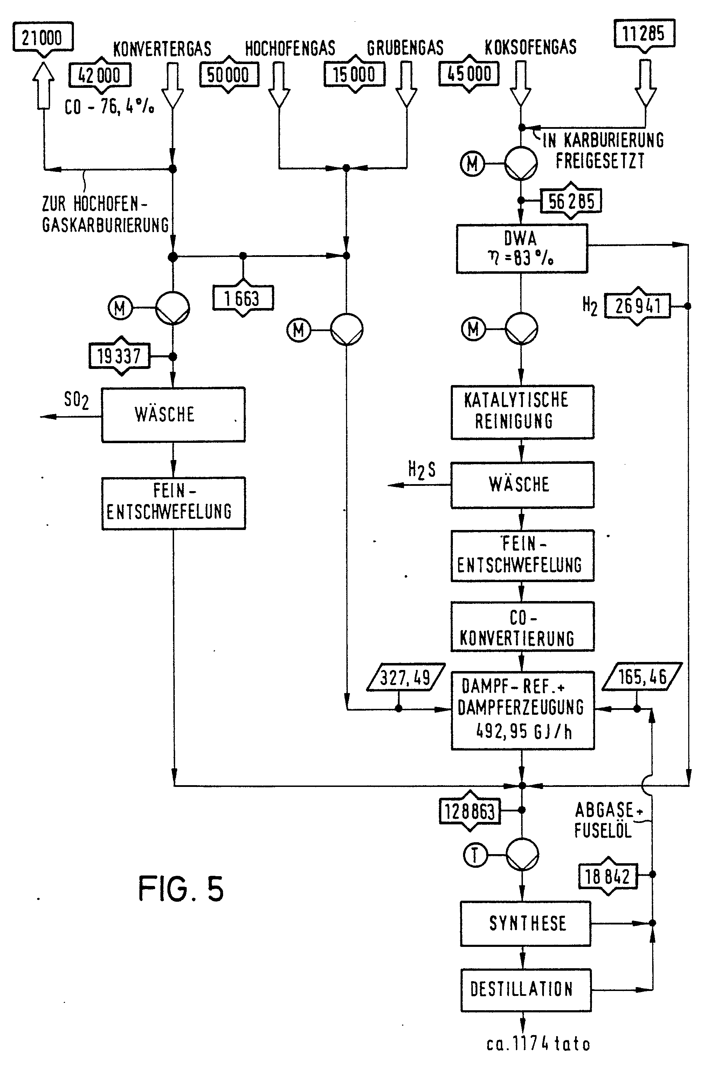

Um die Kosten intensiven Verfahrensschritte CO-Konvertierung und $CO_2$-Wäsche in der Konvertergas-Aufbereitung zu eliminieren, wird vorgeschlagen, einen Teil des Konvertergases zur Hochofengaskarburierung einzusetzen und dadurch weiteres Koksofengas aus der Unterfeuerung der Koksofenbatterien für die Methanolsynthese verfügbar zu machen (Abb. 5). Wird im Unterfeuerungsgas der Koksofenbatterien Konventergas (21 000 m³/h) entsprechend dem Heizwert gegen Koksofengas (11 285 m³/h) ausgetauscht, so steht mehr wasserstoffreiches Koksofengas für die Methanolsynthese zur Verfügung und gleichzeitig entfallen die Aufwendungen für die CO-Konvertierung und $CO_2$-Wäsche.

Der thermische Wirkungsgrad steigt durch diese Maßnahme von 61 % auf 66 % und die Methanolproduktion auf 1174 tato.

**Ansprüche**

1. Verfahren zur Herstellung von Chemierohstoffen wie z.B. Methanol, Ammoniak aus Koksofengas und Hüttengasen wie z.B. Konvertergas, wobei aus diesen Gasen zunächst ein stöchiometrisches Synthesegas derart erzeugt wird, daß Koksofengas und Konvertergas in einem vorgegebenen Volumenverhältnis $H_2$ und CO gemischt und methanisiert werden, welches in einer Synthese zu Chemierohstoffen wie z.B. Methanol, Ammoniak umgewandelt wird, dadurch gekennzeichnet, daß das Koksofengas bzw. aufbereitete Koksofengas mit Konvertergas im Volumenverhältnis $H_2$ und CO von ca. 2 gemischt und dann direkt in einer Methanolsynthese eingesetzt wird.

2. Verfahren nach Anspruch 1, dadurch gekennzeichnet, daß das Koksofengas nach einer Verdichtung über eine Druck-Wechsel-Anlage (DWA) geleitet wird, in der ca. 83% des im Koksofengas enthaltenen Wasserstoffs abgetrennt werden, das Abgas der DWA wird erneut verdichtet und anschließend in einer Reinigungsstufe (Wäsche + katalytsiche Reinigung) von Katalysatorgiften befreit; wonach das so gereinigte Gas konvertiert wird, um Kohlenstoffablagerungen im folgenden Dampf-Reformer zu vermeiden und so eine Schädigung des Katalysators zu verhindern, wobei im Dampfreformer das im Gas enthaltene Methan mit Hilfe von Wasserdampf zu CO, $CO_2$ und $H_2$ aufgespalten, das Spaltgas mit dem in der DWA-Anlage abgetrennten Wasserstoff und mit einer zur Erzeugung eines stöchiometrischen Synthesegases erforderlichen Menge Konvertergas, welches vorher verdichtet und gereinigt (Wäsche und Feinentschwefelung zur Entfernung der Katalysatorgifte) wurde, gemischt und anschließend das Synthesegas auf Synthesedruck verdichtet und in der Methanolsynthese und Destillation zu der gewünschten Produktqualität aufgearbeitet wird.

3. Verfahren nach Anspruch 2, dadurch gekennzeichnet, daß die zur Spaltung des DWA-Abgases im Reformer benötigte Wärmemenge durch Abgas aus Synthese und Destillation sowie überschüssiges Konvertergas gedeckt wird.

4. Verfahren nach den Ansprüchen 1 und 2, dadurch gekenzeichnet, daß der im Koksofengas enthaltene Wasserstoff nicht über eine DWA-Anlage abgetrennt wird, sondern das verdichtete Koksofengas direkt in einer Reinigungsstufe gereinigt, danach konvertiert, das im Gas enthaltene Methan zu CO, $CO_2$ und $H_2$ aufgespalten und das Spaltgas mit einer zur Erzeugung des stöchiometrischen Synthesegases erforderlichen Menge Konvertergas, welches vorher verdichtet und gereinigt wurde, gemischt wird.

5. Verfahren nach Anspruch 1, dadurch gekennzeichnet, daß das Koksofengas nach Verdichtung zunächst von Katalysatorgiften in einer Reinigungsstufe (Wäsche und Kat. Reinigung) befreit , anschließend mit Konvertergas, das vorher komprimiert und in Reinigungsstufen (Wäsche und Feinentschwefelung) von Katalysatorgiften befreit wurde, sowie Spaltgas, das in einem Dampfreformer aus einem Gas, bei dem es sich um 65% des Abblasgases (methanreich) aus der Synthese handelt, das vor der Dampfreformierung noch konvertiert wird, um Kohlenstoffablagerungen im Dampfreformer zu vermeiden, wobei das im Gas enthaltene Methan mit Hilfe von Wasserdampf zu CO, $CO_2$ und $H_2$ aufgespalten wird, gemischt wird, wobei das stöchiometrische Gasgemisch aus Konververtergas, Koksofengas und Spaltgas auf Synthesedruck verdichtet und in der Methanolsynthese und Destillation auf die gewünschte Produktqualität aufgearbeitet wird.

6. Verfahren nach Anspruch 5, dadurch gekennzeichnet, daß zur Beheizung des Dampfreformers überschüssiges Konververtergas und das restliche Abgas aus der Synthese sowie der Destillation, eingesetzt werden.

7. Verfahren nach den Ansprüchen 1 und 2,

**dadurch gekennzeichnet,** daß das gereinigte Koksofengas nicht der CO-Konvertierung unterzogen wird, sondern direkt dem Dampfreformer zur Spaltung zugeführt wird.

8. Verfahren nach einem oder mehreren der vorhergehenden Ansprüche, **dadurch gekennzeichnet,** daß Hochofengas und/oder Grubengas als Heizenergie eingesetzt werden, so daß dann mehr bisher verbranntes Konvertergas für die Methanolsynthese zur Verfügung gestellt wird, wobei jedoch ein erheblicher Anteil des Konvertergases mit Wasserdampf über eine CO-Konvertierung in Wasserstoff umgewandelt wird, um ein stöchiometrisches Gasgemisch aus Koksofengas und Konvertergas für die Methanolsynthese zu erreichen.

9. Verfahren nach Anspruch 8, **dadurch gekennzeichnet,** daß ein Teil des Konvertergases zur Hochofenkarburierung eingesetzt wird, wodurch weiteres Koksofengas aus der Unterfeuerung der Koksofenbatterien für die Methanolsynthese verfügbar wird.

10. Verfahren nach Anspruch 9, **dadurch gekennzeichnet,** daß im Unterfeuerungsgas der Koksofenbatterien Konvertergas entsprechend dem Heizwert gegen Koksofengas ausgetauscht wird, so daß mehr wasserstoffreiches Koksofengas für die Methanolsynthese zur Verfügung steht.

0 200 880

FIG.1

45 000 | KOKSOFENGAS

(M) ⊘

DWA
η=83%

21540

(M) ⊘

KATALYTISCHE
REINIGUNG

H₂S ← WÄSCHE

FEINENT-
SCHWEFELUNG

CO-
KONVERTIERUNG

ca. 37 ← DAMPFEX-
PORT HD | STEAM-REF.+
DAMPFERZEU-
GUNG 437,9GJ/h

(T) ⊘

87567

42507

PURGE + TAILGAS+
FUSELÖL

SYNTHESE

DESTILLATION

ca. 457 tato

# FIG. 2

η th= 48%

⬭ t/h

▱ 10⁶ kJ/h

⬡ m³Vn/h

(T) Dampfturbinen-
Antrieb

(M) Motorantrieb

**FIG. 3 VARIANTE 1**

KOKSOFENGAS — 45 000 — DWA η=83% — WÄSCHE+KAT. REINIGUNG — CO-KONVERTIERUNG — DAMPF-REF. 179,2 GJ/h — 21 540 — ABGASE+FUSELÖL — SYNTHESE — DESTILLATION — ca. 939 tato METHANOL

KONVERTERGAS — 42 000 — WÄSCHE — FEINENT-SCHWEFELUNG — 15 460 — 103 027

**FIG. 3 VARIANTE 2**

KOKSOFENGAS — 45 000 — WÄSCHE+KAT. REINIGUNG — CO-KONVERTIERUNG — DAMPF-REF. 195,9 GJ/h — ABGASE+FUSELÖL — SYNTHESE — DESTILLATION — ca. 903 tato METHANOL

KONVERTERGAS — 42 000 — WÄSCHE — FEINENT-SCHWEFELUNG — 15 108 — 99 695

0 200 880

**FIG. 3 VARIANTE 3**

KOKSOFENGAS — 45 000 — WÄSCHE+KAT. REINIGUNG — CO-KONVERTIERUNG — DAMPF-REF. 128,5 GJ/h — SYNTHESE — DESTILLATION — ca. 702 tato METHANOL

65% ABGASE — ABGASE — ABGASE+FUSELÖL

KONVERTERGAS — 42 000 — WÄSCHE — FEINENT-SCHWEFELUNG

13 704 — 119 004

**FIG. 3 VARIANTE 4**

KOKSOFENGAS — 45 000 — DWA $\eta = 83\%$ — 21 540 $H_2$ — WÄSCHE+KAT. REINIGUNG — DAMPF-REF. 175,5 GJ/h — SYNTHESE — DESTILLATION — ca. 937 tato METHANOL

ABGASE+FUSELÖL

KONVERTERGAS — 42 000 — WÄSCHE — FEINENT-SCHWEFELUNG

15 460 — 102 454

0 200 880

FIG. 4

FIG. 5